# EUROPEAN PATENT SPECIFICATION

(11) **EP 3 452 828 B1**
(45) Date of publication and mention of the grant of the patent: **18.03.2026**
(21) Application number: 17793538.4
(22) Date of filing: 07.05.2017
(51) Int. Cl.: G01N 33/53, G01N 33/68

(54) **SYNAPTIC PROTEIN BIOMARKERS AND DIFFERENTIAL DIAGNOSIS OF ALZHEIMER'S DISEASE AND OTHER NEURODEGENERATIVE DISORDERS**
SYNAPTISCHE PROTEINBIOMARKER UND DIFFERENZIELLE DIAGNOSE VON MORBUS ALZHEIMER UND ANDEREN NEURODEGENERATIVEN ERKRANKUNGEN
BIOMARQUEURS DE PROTÉINES SYNAPTIQUES ET DIAGNOSTIC DIFFÉRENTIEL DE LA MALADIE D'ALZHEIMER ET D'AUTRES TROUBLES NEURODÉGÉNÉRATIFS

(30) Priority: 06.05.2016 US 201662332479 P
(43) Date of publication of application: 13.03.2019
(73) Proprietor: Nanosomix Inc., Aliso Viejo, CA 92656 (US)
(72) Inventor: GOETZL, Edward J., San Francisco, CA 94115 (US)
(74) Representative: Carpmaels & Ransford LLP
(86) International application number: PCT/US2017/031487
(87) International publication number: WO 2017/193115

(56) References cited:
- WO-A1-2014/004424
- WO-A2-2015/130956
- CA-A1- 2 574 727
- US-A1- 2005 244 890
- US-A1- 2005 260 654
- US-A1- 2005 260 697
- US-A1- 2007 037 200
- US-A1- 2008 220 449
- US-A1- 2015 119 278
- ERIN L. ABNER ET AL: "Plasma neuronal exosomal levels of Alzheimer's disease biomarkers in normal aging", ANNALS OF CLINICAL AND TRANSLATIONAL NEUROLOGY, vol. 3, no. 5, 13 April 2016 (2016-04-13), GB, pages 399 - 403, XP055384122, ISSN: 2328-9503, DOI: 10.1002/acn3.309
- CHUN-I SZE ET AL: "Selective regional loss of exocytotic presynaptic vesicle proteins in Alzheimer's disease brains", JOURNAL OF NEUROLOGICAL SCIENCES., vol. 175, no. 2, 1 April 2000 (2000-04-01), NL, pages 81 - 90, XP055634722, ISSN: 0022-510X, DOI: 10.1016/S0022-510X(00)00285-9
- LV LIN-LI ET AL: "CD2AP mRNA in urinary exosome as biomarker of kidney disease", CLINICA CHIMICA ACTA, ELSEVIER BV, AMSTERDAM, NL, vol. 428, 18 October 2013 (2013-10-18), pages 26 - 31, XP028801183, ISSN: 0009-8981, DOI: 10.1016/J.CCA.2013.10.003
- PIA DAVIDSSON ET AL: "Identification of synaptic vesicle, pre- and postsynaptic proteins in human cerebrospinal fluid using liquid-phase isoelectric focusing", ELECTROPHORESIS, vol. 20, 1 March 1999 (1999-03-01), pages 431 - 437, XP055064229
- EDWARD J. GOETZL ET AL: "Decreased synaptic proteins in neuronal exosomes of frontotemporal dementia and Alzheimer's disease", THE FASEB JOURNAL, vol. 30, no. 12, 6 September 2016 (2016-09-06), US, pages 4141 - 4148, XP055569181, ISSN: 0892-6638, DOI: 10.1096/fj.201600816R
- GEBHARDT F M ET AL: "Housekeepers for accurate transcript expression analysis in Alzheimer's disease autopsy brain tissue", ALZHEIMER'S & DEMENTIA: THE JOURNAL OF THE ALZHEIMER'SASSOCIATION, ELSEVIER, NEW YORK, NY, US, vol. 6, no. 6, 1 November 2010 (2010-11-01), pages 465 - 474, XP027455943, ISSN: 1552-5260, [retrieved on 20101031], DOI: 10.1016/J.JALZ.2009.11.002
- REDDY P HEMACHANDRA ET AL: "Differential loss of synaptic proteins in Alzheimer's disease: implications for synaptic dysfunction", JOURNAL OF ALZHEIMER`S DISEASE, IOS PRESS, NL, vol. 7, no. 2, 1 April 2005 (2005-04-01), pages 103 - 117, 173, XP009172020, ISSN: 1387-2877
- SCHLAF GERALD ET AL: "Determination of synapsin I and synaptophysin in body fluids by two-site enzyme-linked immunosorbent assays", JOURNAL OF IMMUNOLOGICAL METHODS, vol. 213, no. 2, 1 June 1998 (1998-06-01), NL, pages 191 - 199, XP093099009, ISSN: 0022-1759, DOI: 10.1016/S0022-1759(98)00027-1

## Description

### RELATED APPLICATIONS

This application claims priority to the U.S. Provisional Patent Application Serial No. 62/332,479, filed on May 6, 2016.

### FIELD OF THE INVENTION

The present disclosure relates to synaptic protein biomarkers and diagnostic and prognostic methods for Alzheimer's disease and other neurodegenerative disorders. The disclosure also provides compositions for detecting the synaptic protein biomarkers as well as compositions and methods useful for early diagnosis and/or treatment of Alzheimer's disease and other neurodegenerative disorders. The invention provides methods for detecting biomarkers in vesicles (e.g., exosomes) isolated from a biological sample. The scope of the invention is defined by the appended claims. Embodiments and descriptions no longer falling under the scope of the appended claims are considered merely as examples suitable for understanding the invention.

### BACKGROUND OF THE INVENTION

More than 5.4 million Americans and 35 million people worldwide have Alzheimer's disease, the most common form of dementia. Currently, the only definitive way to diagnose Alzheimer's disease is by direct examination of brain tissue after a patient dies. Doctors use brain imaging, evaluation of behavior, psychiatric tests, and other means to diagnose the disease in the patients suspected of having Alzheimer's disease, but none are highly accurate, and many are costly or not practical.

Therefore, there is a need in the art for biomarkers and methods for diagnosing Alzheimer's disease and other neurodegenerative disorders. Additionally, there is a need in the art for compositions for detecting the biomarkers as well as compositions and methods useful for treating Alzheimer's disease and other neurodegenerative disorders. The present disclosure meets this need by providing accurate, noninvasive methods for diagnosing Alzheimer's disease and other neurodegenerative disorders. The present disclosure further provides novel methods, assays, biomarkers, kits, and compositions for diagnosing, prognosing, predicting, and treating Alzheimer's disease and other neurodegenerative disorders.

Schlaf, Gerald et al, Journal of Immunological Methods 213 (2) 1998, pp 191-199, refers to a sandwich ELISA assay designed for the detection of synapsin and synaptophysin in body fluid samples.

### SUMMARY OF THE INVENTION

The present disclosure provides methods of diagnosing or prognosing a neurodegenerative disorder in a subject, identifying a subject at risk of a neurodegenerative disorder, or prescribing a therapeutic regimen or predicting benefit from therapy in a subject having a neurodegenerative disorder, comprising: assaying the level of one or more biomarkers in a biological sample from the subject; and diagnosing or prognosing a neurodegenerative disorder in a subject, identifying a subject at risk of a neurodegenerative disorder, or prescribing a therapeutic regimen or predicting benefit from therapy in a subject having a neurodegenerative disorder based on the levels of the biomarker, wherein at least one of the one or more biomarkers are selected from the group consisting of synaptophysin, synaptopodin, synaptotagmin, neurogranin, and human growth associated protein 43 (GAP43). In some embodiments, the level of the one or more biomarkers in the biological sample is compared to the level of one or more biomarkers in a control sample and wherein the level of the one or more biomarkers of the biological sample is elevated compared to the control sample. In some embodiments, the level of the one or more biomarkers in the biological sample is compared to the level of one or more biomarkers in a control sample and wherein the level of the one or more biomarkers of the biological sample is decreased compared to the control sample. In other embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In other embodiments, the biological sample is selected from the group consisting of whole blood, serum, plasma, urine, interstitial fluid, peritoneal fluid, cervical swab, tears, saliva, buccal swab, skin, brain tissue, and cerebrospinal fluid. In other embodiments, the method further comprises isolating vesicles from the biological samples. In other embodiments, the method further comprises incubating or treating the biological sample with a lytic agent. In other embodiments, the vesicles are selected from the group consisting of exosomes, microparticles, microvesicles, nanosomes, extracellular vesicles, and ectosomes. In other embodiments, the method further comprises isolating exosomes from the biological sample. In certain embodiments, the isolated exosomes are selected from the group consisting of neuron-derived exosomes, astrocyte-derived exosomes, oliogodendrocyte-derived exosomes, and microglia-derived exosomes. In other embodiments, the level of one or more biomarkers is the protein, mRNA, or miRNA level of the one or more biomarker. In some aspects, the methods of the present disclosure further comprise predicting the movement from preclinical to the manifestation of a neurodegenerative disorder. In other aspects, the methods of the present disclosure further comprise predicting outcome or worsening of the neurodegenerative disorder. In yet other aspects, the methods of the present disclosure comprise preventing Alzheimer's disease. In other embodiments, the method further comprises measuring the level of one or more biomarkers in the biological sample, wherein at least one of the one or more biomarkers are selected from the group consisting of Tau, phosphorylated Tau, Aβ1-42, TDP-43, α-synuclein, SOD-1, FUS, FKBP51, IRS-1, phosphorylated IRS-1, cathepsin D (CTSD), type 1 lysosome-associated membrane protein (LAMP1), ubiquitinylated proteins (UBP), heat-shock protein 70 (HSP70), neuron-specific enolase (NSE), neurofilament light chain (NFL) , low-density lipoprotein receptor-related protein 6 (LPR6), heat-shock factor-1 (HSF1), and repressor element 1-silencing transcription factor (REST), CD9, CD63, CD81, and CD171.

The present disclosure provides methods comprising: obtaining a biological sample comprising vesicles from a subject; isolating vesicles from the biological sample; and detecting one or more biomarkers in the biological sample, wherein at least one of the one or more biomarkers are selected from the group consisting of synaptophysin, synaptopodin, synaptotagmin, neurogranin, and human growth associated protein 43 (GAP43). In certain embodiments, the biological sample is selected from the group consisting of whole blood, serum, plasma, urine, interstitial fluid, peritoneal fluid, cervical swab, tears, saliva, buccal swab, skin, brain tissue, and cerebrospinal fluid. In other embodiments, the method further comprises incubating or treating the biological sample with a lytic agent. In other embodiments, the vesicles are selected from the group consisting of exosomes, microparticles, microvesicles, nanosomes, extracellular vesicles, and ectosomes. In other embodiments, the method further comprises isolating exosomes from the biological sample. In certain embodiments, the isolated exosomes are selected from the group consisting of neuron-derived exosomes, astrocyte-derived exosomes, oliogodendrocyte-derived exosomes, and microglia-derived exosomes. In some aspects, the methods of the present invention further comprise predicting the movement from preclinical to the manifestation of a neurodegenerative disorder. In other aspects, the methods of the present invention further comprise predicting outcome or worsening of the neurodegenerative disorder. In yet other aspects, the methods of the present invention comprise preventing Alzheimer's disease. In other embodiments, the method further comprises detecting one or more biomarkers in the biological sample, wherein at least one of the one or more biomarkers are selected from the group consisting of Tau, phosphorylated Tau, Aβ1-42, TDP-43, α-synuclein, SOD-1, FUS, FKBP51, IRS-1, phosphorylated IRS-1, cathepsin D (CTSD), type 1 lysosome-associated membrane protein (LAMP1), ubiquitinylated proteins (UBP), heat-shock protein 70 (HSP70), neuron-specific enolase (NSE), neurofilament light chain (NFL) , low-density lipoprotein receptor-related protein 6 (LPR6), heat-shock factor-1 (HSF1), and repressor element 1-silencing transcription factor (REST), CD9, CD63, CD81, and CD171.

The present disclosure also provides methods of diagnosing or prognosing a neurodegenerative disorder in a subject, identifying a subject at risk of a neurodegenerative disorder, or prescribing a therapeutic regimen or predicting benefit from therapy in a subject having a neurodegenerative disorder, comprising: isolating vesicles from a biological sample obtained from the subject; and determining the level of one or more biomarkers in the vesicles; wherein an elevated level of the one or more biomarkers in the sample compared to the level of the one or more biomarkers in a control sample is an indication of a neurodegenerative disorder, wherein at least one of the one or more biomarkers is selected from the group consisting of synaptophysin, synaptopodin, synaptotagmin, neurogranin, and GAP43. In some embodiments, the level of the one or more biomarkers in the biological sample is decreased compared to the control sample. In other embodiments, the vesicles are selected from the group consisting of exosomes, microparticles, microvesicles, nanosomes, extracellular vesicles, and ectosomes. In some embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In other embodiments, the biological sample is selected from the group consisting of whole blood, serum, plasma, urine, interstitial fluid, peritoneal fluid, cervical swab, tears, saliva, buccal swab, skin, brain tissue, and cerebrospinal fluid. In still other embodiments, the isolated exosomes are selected from the group consisting of neuron-derived exosomes, astrocyte-derived exosomes, oliogodendrocyte-derived exosomes, and microglia-derived exosomes. In other embodiments, the isolating vesicles from a biological sample comprises: contacting the biological sample with an agent under conditions wherein a vesicle present in said biological sample binds to said agent to form a vesicle-agent complex; and isolating said vesicle from said vesicle-agent complex to obtain a sample containing said vesicle, wherein the purity of vesicles present in said sample is greater than the purity of vesicles present in said biological sample. In other embodiments, the isolating vesicles from a biological sample comprises: isolating vesicles from said biological sample to obtain a vesicle sample; contacting the vesicle sample with an agent under conditions wherein a vesicle present in said vesicle sample binds to said agent to form a vesicle-agent complex; and isolating said vesicle from said vesicle-agent complex to obtain a sample containing said vesicle, wherein the purity of vesicles present in said sample is greater than the purity of vesicles present in said biological sample. In certain aspects, the agent is an antibody, a lectin, a ligand, a soluble receptor, a binding protein, or an oligonucleotide. In other aspects, the antibody is a polyclonal or monoclonal antibody. In yet other aspects, the antibody is a monoclonal NCAM antibody. In other aspects, the antibody is a monoclonal anti-human NCAM antibody. In yet other aspects, the antibody is a monoclonal CD171 antibody. In other aspects, the antibody is a monoclonal anti-human CD171 antibody. In other aspects, the antibody is a monoclonal CD9 antibody. In other aspects, the antibody is a monoclonal CD63 antibody. In other aspects, the antibody is a monoclonal CD81 antibody. In other aspects, the antibody is a neuron-specific enolase antibody. In other aspects, the antibody is a monoclonal neuron-specific enolase antibody. In other embodiments, the vesicles are selected from the group consisting of exosomes, microparticles, microvesicles, nanosomes, extracellular vesicles, and ectosomes. In other embodiments, the level of one or more biomarkers is the protein, mRNA, or miRNA level of the one or more biomarker.

The present disclosure provides methods of diagnosing or prognosing a neurodegenerative disorder in a subject, identifying a subject at risk of a neurodegenerative disorder, or prescribing a therapeutic regimen or predicting benefit from therapy in a subject having a neurodegenerative disorder, comprising: obtaining a biological sample from the subject; applying an antibody specific for vesicles to the sample, wherein the presence of the vesicle creates an antibody-vesicle complex; isolating the antibody-vesicle complex; assaying a level of one or more biomarkers in the antibody-vesicle complex; and diagnosing or prognosing a neurodegenerative disorder in a subject, identifying a subject at risk of a neurodegenerative disorder, or prescribing a therapeutic regimen or predicting benefit from therapy in a subject having a neurodegenerative disorder based on the levels of the one or more biomarkers, wherein at least one of the biomarkers are selected from the group consisting of synaptophysin, synaptopodin, synaptotagmin, neurogranin, and GAP43. In some embodiments, the antibody-vesicle complex is created on a solid phase. In yet other embodiments, the methods further comprise releasing the vesicle from the antibody-vesicle complex. In other embodiments, the vesicle is released using a competing peptide that competes for the binding of the selection antibody used in the methods of the present invention. In certain embodiments, the solid phase is non-magnetic beads, magnetic beads, agarose, or sepharose. In other embodiments, the vesicle is released by exposing the antibody-vesicle complex to low pH between 3.5 and 1.5. In yet other embodiments, the released vesicle is neutralized by adding a high pH solution. In other embodiments, the released vesicle is lysed by incubating the released vesicles with a lysis solution. In still other embodiments, the lysis solution contains inhibitors for proteases and phosphatases. In other embodiments, the levels of the one or more biomarkers are normalized by the number of vesicles or values of vesicle biomarkers. In other embodiments, the vesicles are selected from the group consisting of exosomes, microparticles, microvesicles, nanosomes, extracellular vesicles, and ectosomes. In certain embodiments, the antibody is a polyclonal or monoclonal antibody. In other embodiments, the antibody is a monoclonal NCAM antibody. In other embodiments, the antibody is a monoclonal anti-human NCAM antibody. In yet other aspects, the antibody is a monoclonal CD171 antibody. In other aspects, the antibody is a monoclonal anti-human CD171 antibody. In other aspects, the antibody is a monoclonal CD9 antibody. In other aspects, the antibody is a monoclonal CD63 antibody. In other aspects, the antibody is a monoclonal CD81 antibody. In other aspects, the antibody is a neuron-specific enolase antibody. In other aspects, the antibody is a monoclonal neuron-specific enolase antibody. In some embodiments, the exosomes are selected from the group consisting of neuron-derived exosomes, astrocyte-derived exosomes, oliogodendrocyte-derived exosomes, and microglia-derived exosomes. In yet other embodiments, the biological sample is selected from the group consisting of whole blood, serum, plasma, urine, interstitial fluid, peritoneal fluid, cervical swab, tears, saliva, buccal swab, skin, brain tissue, and cerebrospinal fluid. In some embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In other embodiments, the level of one or more biomarkers is the protein, mRNA, or miRNA level of the one or more biomarker.

The present disclosure provides sets of biomarkers for assessing neurodegenerative disorder status of a subject comprising one or more biomarkers, wherein the levels of the biomarkers in the set are assayed; and wherein the biomarker level determines the neurodegenerative disorder status of the subject with at least 40% specificity, wherein the at least one or more of the set of biomarkers are selected from the group consisting of synaptophysin, synaptopodin, synaptotagmin, neurogranin, and GAP43. In some embodiments, the biomarker level determines the neurodegenerative disorder status of the subject with at least 40%, 50%, 60%, 70%, 75%, 80%, 85%, 90%, 95%, 99%, or 100% specificity. In some embodiments, the biological sample is selected from the group consisting of whole blood, serum, plasma, urine, interstitial fluid, peritoneal fluid, cervical swab, tears, saliva, buccal swab, skin, cerebrospinal fluid. In other embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In yet other embodiments, the methods further comprise assaying the levels of the biomarkers in vesicles from the sample. In other embodiments, the vesicles are selected from the group consisting of exosomes, microparticles, microvesicles, nanosomes, extracellular vesicles, and ectosomes. In other embodiments, the sets of biomarkers of the present invention further comprise one or more biomarkers selected from the group consisting of Tau, phosphorylated Tau, Aβ1-42, TDP-43, α-synuclein, SOD-1, FUS, FKBP51, IRS-1, phosphorylated IRS-1, cathepsin D (CTSD), type 1 lysosome-associated membrane protein (LAMP1), ubiquitinylated proteins (UBP), heat-shock protein 70 (HSP70), neuron-specific enolase (NSE), neurofilament light chain (NFL), low-density lipoprotein receptor-related protein 6 (LPR6), heat-shock factor-1 (HSF1), and repressor element 1-silencing transcription factor (REST), CD9, CD63, CD81, and CD171.

The present disclosure also provides kits for diagnosing or prognosing a neurodegenerative disorder in a subject, identifying a subject at risk of a neurodegenerative disorder, or prescribing a therapeutic regimen or predicting benefit from therapy in a subject having a neurodegenerative disorder, the kit comprising one or more agents which specifically binds vesicles, one or more agents which specifically bind a biomarker, one or more containers for collecting and or holding the biological sample, and an instruction for its use, wherein the neurodegenerative disorder is associated with altered biomarker levels and wherein the biomarker is selected from the group consisting of synaptophysin, synaptopodin, synaptotagmin, neurogranin, and GAP43. In other embodiments, the vesicles are selected from the group consisting of exosomes, microparticles, microvesicles, nanosomes, extracellular vesicles, and ectosomes. In some embodiments, the agents are polyclonal or monoclonal antibodies. In other embodiments, the antibodies are a monoclonal NCAM antibody. In other embodiments, the antibody is a monoclonal anti-human NCAM antibody. In yet other aspects, the antibody is a monoclonal CD171 antibody. In other aspects, the antibody is a monoclonal anti-human CD171 antibody. In other aspects, the antibody is a monoclonal CD9 antibody. In other aspects, the antibody is a monoclonal CD63 antibody. In other aspects, the antibody is a monoclonal CD81 antibody. In other aspects, the antibody is a neuron-specific enolase antibody. In other aspects, the antibody is a monoclonal neuron-specific enolase antibody. In certain embodiments, the exosomes are selected from the group consisting of neuron-derived exosomes, astrocyte-derived exosomes, oliogodendrocyte-derived exosomes, and microglia-derived exosomes. In other embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In yet other embodiments, the biological sample is selected from the group consisting of whole blood, serum, plasma, urine, interstitial fluid, peritoneal fluid, cervical swab, tears, saliva, buccal swab, skin, cerebrospinal fluid. In other embodiments, the kits further comprise a computer model or algorithm for analyzing the biomarker level in the sample.

The present disclosure also provides kits for diagnosing or prognosing a neurodegenerative disorder in a subject, identifying a subject at risk of a neurodegenerative disorder, or prescribing a therapeutic regimen or predicting benefit from therapy in a subject having a neurodegenerative disorder, the kit comprising one or more agents which specifically binds vesicles, one or more probes or primers for detecting biomarker mRNA or miRNA, one or more containers for collecting and or holding the biological sample, and an instruction for its use, wherein the neurodegenerative disorder is associated with altered biomarker levels and wherein the biomarker is selected from the group consisting of synaptophysin, synaptopodin, synaptotagmin, neurogranin, and GAP43. In other embodiments, the vesicles are selected from the group consisting of exosomes, microparticles, microvesicles, nanosomes, extracellular vesicles, and ectosomes. In some embodiments, the agents are polyclonal or monoclonal antibodies. In other embodiments, the exosomes are selected from the group consisting of neuron-derived exosomes, astrocyte-derived exosomes, oliogodendrocyte-derived exosomes, and microglia-derived exosomes. In yet other embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In still other embodiments, the biological sample is selected from the group consisting of whole blood, serum, plasma, urine, interstitial fluid, peritoneal fluid, cervical swab, tears, saliva, buccal swab, skin, cerebrospinal fluid. In other embodiments, the kits further comprise a computer model or algorithm for analyzing the biomarker level in the sample. In some embodiments, the kits of the present invention further comprise one or more agents which specifically bind to one or more biomarkers selected from the group consisting of Tau, phosphorylated Tau, Aβ1-42, TDP-43, α-synuclein, SOD-1, FUS, FKBP51, IRS-1, phosphorylated IRS-1, cathepsin D (CTSD), type 1 lysosome-associated membrane protein (LAMP1), ubiquitinylated proteins (UBP), heat-shock protein 70 (HSP70), neuron-specific enolase (NSE), neurofilament light chain (NFL), low-density lipoprotein receptor-related protein 6 (LPR6), heat-shock factor-1 (HSF1), and repressor element 1-silencing transcription factor (REST), CD9, CD63, CD81, and CD171.

In other embodiments, the present disclosure provides methods of diagnosing a neurodegenerative disorder in a subject, comprising the steps of: (i) obtaining a test biological sample containing vesicles from the subject, (ii) measuring the level of one or more biomarkers in the test biological sample, (iii) comparing the level of the one or more biomarkers in the test biological sample to a control level of the one or more biomarkers in a control biological sample, and (iv) determining the subject has a neurodegenerative disorder by detecting an increased or decreased level of the one or more biomarkers in the test biological sample, relative to the control biological sample, wherein at least one of the one or more biomarkers is selected from the group consisting of synaptophysin, synaptopodin, synaptotagmin, neurogranin, and GAP43. In other embodiments, the vesicles are selected from the group consisting of exosomes, microparticles, microvesicles, nanosomes, extracellular vesicles, and ectosomes. In some embodiments, the exosomes are selected from the group consisting of neuron-derived exosomes, astrocyte-derived exosomes, oliogodendrocyte-derived exosomes, and microglia-derived exosomes. In yet other embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In still other embodiments, the biological sample is selected from the group consisting of whole blood, serum, plasma, urine, interstitial fluid, peritoneal fluid, cervical swab, tears, saliva, buccal swab, skin, cerebrospinal fluid. In other embodiments, the method further comprises isolating vesicles from the biological sample. In other embodiments, the method further comprises incubating or treating the biological sample with a lytic agent. In other embodiments, the vesicles are selected from the group consisting of exosomes, microparticles, microvesicles, nanosomes, extracellular vesicles, and ectosomes. In some embodiments a decrease in the level of one or more biomarkers of the present invention is indicative of a first neurodegenerative disorder and a decrease in the level of the same one or more biomarkers is indicative of a second neurodegenerative disorder.

The present invention provides in vitro methods for analyzing a sample from a subject comprising the steps of: (i) obtaining a biological sample comprising vesicles from the subject, (ii) isolating the vesicles from the biological sample and measuring the level of one or more biomarkers in the biological sample, and (iii) comparing the level of the one or more biomarkers in the biological sample to a control level of the one or more biomarkers in a control biological sample, wherein the subject has been diagnosed or suspected of having Alzheimer's disease or another neurodegenerative disorder, wherein the biological sample is obtained from blood, and wherein at least one of the one or more biomarkers is synaptophysin. In other embodiments, the method further comprises diagnosing or prognosing a neurodegenerative disorder in the subject, identifying risk of a neurodegenerative disorder in the subject, or prescribing a therapeutic regimen or predicting benefit from therapy for the subject having or suspected of having a neurodegenerative disorder. In some embodiments, the level of the one or more biomarkers in the biological sample is compared to the level of one or more biomarkers in a control sample and wherein the level of the one or more biomarkers of the biological sample is elevated compared to the control sample. In some embodiments, the level of the one or more biomarkers in the biological sample is compared to the level of one or more biomarkers in a control sample and wherein the level of the one or more biomarkers of the biological sample is decreased compared to the control sample. In other embodiments, the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease. In other embodiments, the method further comprises incubating or treating the biological sample with a lytic agent. In other embodiments, the vesicles are selected from the group consisting of exosomes, microparticles, microvesicles, nanosomes, extracellular vesicles, and ectosomes. In other embodiments, the method further comprises isolating exosomes from the biological sample. In certain embodiments, the isolated exosomes are selected from the group consisting of neuron-derived exosomes, astrocyte-derived exosomes, oliogodendrocyte-derived exosomes, and microglia-derived exosomes. In other embodiments, the methods of the present invention further comprise a computer model or algorithm for analyzing the one or more biomarker level in the sample. In other embodiments, the level of one or more biomarkers is the protein, mRNA, or miRNA level of the one or more biomarker.

In other embodiments, the present disclosure provides methods for diagnosing or prognosing a neurodegenerative disorder in a subject, comprising the steps of: (a) providing a sample comprising vesicles obtained from a subject; (b) assessing the level of one or more biomarkers comprising synaptophysin, synaptopodin, synaptotagmin, neurogranin, and GAP43 in the sample; (c) comparing the synaptophysin, synaptopodin, synaptotagmin, neurogranin, and/or GAP43 levels in the sample with synaptophysin, synaptopodin, synaptotagmin, neurogranin, and/or GAP43 levels in a normal control; and (d) determining whether the subject has or is likely to have a neurodegenerative disorder in accordance with the result of step (c). In certain aspects, a subject with synaptophysin, synaptopodin, synaptotagmin, neurogranin, and GAP43 levels in the sample that are lower than those in the normal control has or is likely to have Alzheimer's disease. In other aspects, a subject with synaptophysin, synaptopodin, synaptotagmin, or neurogranin levels in the sample that are lower than those in the normal control has or is likely to have frontotemporal dementia (FTD). In one embodiment, the level of synaptophysin, synaptopodin, synaptotagmin, neurogranin, and GAP43 is assessed by ELISA. In other embodiments, the method further comprises isolating vesicles from the biological sample. In other embodiments, the method further comprises incubating or treating the biological sample with a lytic agent. In certain embodiments, the vesicles are selected from the group consisting of exosomes, microparticles, microvesicles, nanosomes, extracellular vesicles, and ectosomes.

These and other embodiments of the present invention will readily occur to those of skill in the art in light of the disclosure herein, and all such embodiments are specifically contemplated.

Each of the limitations of the invention can encompass various embodiments of the invention. It is, therefore, anticipated that each of the limitations of the invention involving any one element or combinations of elements can be included in each aspect of the invention. This invention is not limited in its application to the details of construction and the arrangement of components set forth in the following description. The invention is capable of other embodiments and of being practiced or of being carried out in various ways. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items. It must be noted that as used herein and in the appended claims, the singular forms "a," "an," and "the" include plural references unless context clearly dictates otherwise.

### BRIEF DESCRIPTION OF THE DRAWINGS

Figures 1A-1F set forth data showing levels of neural-derived plasma exosomal synaptic proteins in patients with Alzheimer's disease, frontotemporal dementia, and matched cognitively normal case controls.
Figure 2 sets forth data showing ROC plots depicting distinctions between Alzheimer's disease and matched cognitively normal case controls for neural-derived plasma exosomal synaptic protein levels.
Figure 3 sets forth data showing ROC plots depicting distinctions between frontotemporal dementia and matched cognitively normal case controls for neural-derived plasma exosomal synaptic protein levels.
Figure 4 sets forth data showing ROC plots depicting distinctions between Alzheimer's disease and frontotemporal dementia for neural-derived plasma exosomal synaptic protein levels.
Figures 5A-5E set forth data showing sequential levels of neural-derived plasma exosomal synaptic proteins in patients with Alzheimer's disease or frontotemporal dementia measured first at a time of normal cognition and later at a time after diagnosis of dementia.

### DESCRIPTION OF THE INVENTION

It is to be understood that the invention is not limited to the particular methodologies, protocols, cell lines, assays, and reagents described herein, as these may vary. It is also to be understood that the terminology used herein is intended to describe particular embodiments of the present invention, and is in no way intended to limit the scope of the present invention as set forth in the appended claims.

It must be noted that as used herein and in the appended claims, the singular forms "a," "an," and "the" include plural references unless context clearly dictates otherwise. Thus, for example, a reference to "a fragment" includes a plurality of such fragments, a reference to an "antibody" is a reference to one or more antibodies and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. Nothing herein is to be construed as an admission that the invention is not entitled to antedate such disclosure by virtue of prior invention.

The practice of the present invention will employ, unless otherwise indicated, conventional methods of chemistry, biochemistry, molecular biology, cell biology, genetics, immunology and pharmacology, within the skill of the art. Such techniques are explained fully in the literature. See, e.g., Gennaro, A.R., ed. (1990) Remington's Pharmaceutical Sciences, 18th ed., Mack Publishing Co.; Colowick, S. et al., eds., Methods In Enzymology, Academic Press, Inc.; Handbook of Experimental Immunology, Vols. I-IV (D.M. Weir and C.C. Blackwell, eds., 1986, Blackwell Scientific Publications); Maniatis, T. et al., eds. (1989) Molecular Cloning: A Laboratory Manual, 2nd edition, Vols. I-III, Cold Spring Harbor Laboratory Press; Ausubel, F. M. et al., eds. (1999) Short Protocols in Molecular Biology, 4th edition, John Wiley & Sons; Ream et al., eds. (1998) Molecular Biology Techniques: An Intensive Laboratory Course, Academic Press); PCR (Introduction to Biotechniques Series), 2nd ed. (Newton & Graham eds., 1997, Springer Verlag).

The present invention relates, in part, to the discovery that exosomal biomarkers can be assayed to identify subjects having or likely to develop neurodegenerative disorders, including, for example, Alzheimer's disease (AD), multiple sclerosis (MS), and frontotemporal dementia (FTD).

The present invention is based, in part, on the discovery of unexpected decreases in certain biomarkers in neuron-derived exosomes present in the circulation of subjects having neurodegenerative disease (e.g., Alzheimer's disease). The present invention demonstrates that exosomal levels of these biomarkers may be assayed to diagnose a neurodegenerative disorder in a subject having a neurodegenerative disease. The present invention further shows that measurement of certain biomarkers in neuron-derived exosomes from a subject may be used to predict the subsequent development of a neurodegenerative disease (e.g., identify a subject at risk of developing a neurodegenerative disorder).

The present disclosure also provides compositions for use in the methods described herein. Such compositions may include small molecule compounds; peptides and proteins including antibodies or functionally active fragments thereof; and polynucleotides including small interfering ribonucleic acids (siRNAs), micro-RNAs (miRNAs), ribozymes, and anti-sense sequences. (See, e.g., Zeng (2003) Proc Natl Acad Sci USA 100:9779-9784; and Kurreck (2003) Eur J Biochem 270:1628-1644.)

The present disclosure further provides kits for diagnosing or prognosing a neurodegenerative disorder in a subject, identifying a subject at risk of a neurodegenerative disorder, or prescribing a therapeutic regimen or predicting benefit from therapy in a subject having a neurodegenerative disorder. In these embodiments, the kits comprise one or more antibodies which specifically binds exosomes, one or more antibodies which specifically bind a biomarker, one or more containers for collecting and or holding the biological sample, and an instruction for the kits use.

The section headings are used herein for organizational purposes only, and are not to be construed as in any way limiting the subject matter described herein.

### Biological Sample

The present disclosure provides biomarkers and diagnostic and prognostic methods for Alzheimer's disease and other neurodegenerative disorders. Biomarkers levels are determined in a biological sample obtained from a subject. In some embodiments, the biological sample of the invention can be obtained from blood. In some embodiments, about 1-10 mL of blood is drawn from a subject. In other embodiments, about 10 -50 mL of blood is drawn from a subject. Blood can be drawn from any suitable area of the body, including an arm, a leg, or blood accessible through a central venous catheter. In some embodiments, blood is collected following a treatment or activity. For example, blood can be collected following a medical exam. The timing of collection can also be coordinated to increase the number and/or composition of exosomes present in the sample. For example, blood can be collected following exercise or a treatment that induces vascular dilation.

Blood may be combined with various components following collection to preserve or prepare samples for subsequent techniques. For example, in some embodiments, blood is treated with an anticoagulant, a cell fixative, a protease inhibitor, a phosphatase inhibitor, a protein, a DNA, or an RNA preservative following collection. In some embodiments, blood is collected via venipuncture using vacuum collection tubes containing an anticoagulant such as EDTA or heparin. Blood can also be collected using a heparin-coated syringe and hypodermic needle. Blood can also be combined with components that will be useful for cell culture. For example, in some embodiments, blood is combined with cell culture media or supplemented cell culture media (e.g., cytokines).

Biological samples can also be obtained from other sources known in the art, including whole blood, serum, plasma, urine, interstitial fluid, peritoneal fluid, cervical swab, tears, saliva, buccal swab, skin, cerebrospinal fluid, or other tissues including, for example, brain tissues.

### Enrichment or Isolation of Vesicles (Exosomes, Microparticles, Microvesicles, Nanosomes, Extracellular Vesicles, and Ectosomes)

Samples can be enriched for vesicles through positive selection, negative selection, or a combination of positive and negative selection. In some embodiments, vesicles are directly captured. In other embodiments, blood cells are captured and vesicles are collected from the remaining biological samples. In some embodiments, the vesicles enriched in the biological samples are exosomes, microparticles, microvesicles, nanosomes, extracellular vesicles, or ectosomes. In some embodiments, the vesicles enriched in the biological samples are neuron-derived exosomes, astrocyte-derived exosomes, oliogodendrocyte-derived exosomes, or microglia-derived exosomes.

Samples can also be enriched for vesicles based on differences in the biochemical properties of vesicles. For example, samples can be enriched for vesicles based on antigen, nucleic acid, metabolic, gene expression, or epigenetic differences. In some of the embodiments based on antigen differences, antibody-conjugated magnetic or paramagnetic beads in magnetic field gradients or fluorescently labeled antibodies with flow cytometry are used. In some of the embodiments based on nucleic acid differences, flow cytometry is used. In some of the embodiments based on metabolic differences, dye uptake/exclusion measured by flow cytometry or another sorting technology is used. In some of the embodiments based on gene expression, cell culture with cytokines is used. Samples can also be enriched for vesicles based on other biochemical properties known in the art. For example, samples can be enriched for vesicles based on pH or motility. Further, in some embodiments, more than one method is used to enrich for vesicles. In other embodiments, samples are enriched for vesicles using antibodies, ligands, or soluble receptors.

In other embodiments, surface markers are used to positively enrich vesicles in the sample. In some embodiments, the vesicles are exosomes, microparticles, microvesicles, nanosomes, extracellular vesicles, or ectosomes. In other embodiments, NCAM, CD171, CD9, CD63, CD81, neuron-specific enolase, diverse neuron or astrocyte adhesive proteins, microglial CD18/11, or CD3 T cell membrane cell surface markers are used to enrich for exosomes. In some embodiments, cell surface markers that are not found on vesicles populations are used to negatively enrich vesicles by depleting cell populations. Flow cytometry sorting may also be used to further enrich for exosomes using cell surface markers or intracellular or extracellular markers conjugated to fluorescent labels. Intracellular and extracellular markers may include nuclear stains or antibodies against intracellular or extracellular proteins preferentially expressed in vesicles. Cell surface markers may include antibodies against cell surface antigens that are preferentially expressed on exosomes (e.g., NCAM). In some embodiments, the cell surface marker is a neuron-derived exosome surface marker, including, for example, NCAM or CD171. In some embodiments, a monoclonal NCAM, CD9, CD63, CD81, neuron-specific enolase or CD171 antibody is used to enrich or isolate exosomes from the sample. In certain aspects, the NCAM, CD9, CD63, CD81, neuron-specific enolase or CD171 antibody is biotinylated. In this embodiment, biotinylated NCAM or CD171 antibody can form an antibody-exosome complex that can be subsequently isolated using streptavidin-agarose resin or beads. In other embodiments, the NCAM, CD9, CD63, CD81, neuron-specific enolase or CD171 antibody is a monoclonal anti-human NCAM, CD9, CD63, CD81, neuron-specific enolase or CD171 antibody.

In some embodiments, enriched vesicles from the biological sample are subsequently enriched for a specific type of vesicle. For example, the biological sample is enriched for exosomes and then the enriched exosomes are subsequently enriched for neural-derived exosomes. In some embodiments, the biological sample is enriched for individual neural cell sources of vesicles. In certain aspects, the neural cell sources of vesicles are microglia, neurons, or astrocytes.

In other embodiments, vesicles are isolated or enriched from a biological sample comprising: contacting a biological sample with an agent under conditions wherein a vesicle present in said biological sample binds to said agent to form a vesicle-agent complex; and isolating said vesicle from said vesicle-agent complex to obtain a sample containing said vesicle, wherein the purity of vesicles present in said sample is greater than the purity of vesicles present in said biological sample. In certain embodiments, the agent is an antibody or a lectin. Lectins useful for forming a vesicle-lectin complex are described in U.S. Patent Application Publication No. 2012/0077263. In some embodiments, the vesicle is an exosome, a microparticle, a microvesicle, nanosomes, extracellular vesicles, or an ectosome. In some embodiments, the exosomes are neuron-derived exosomes, astrocyte-derived exosomes, oliogodendrocyte-derived exosomes, or microglia-derived exosomes. In some embodiments, multiple isolating or enriching steps are performed. In certain aspects of the present embodiment, a first isolating step is performed to isolate exosomes from a blood sample and a second isolating step is performed to isolate neural-derived exosomes from other exosomes. In other embodiments, the vesicle portion of the vesicle-agent complex is lysed using a lysis reagent and the protein levels of the lysed vesicle are assayed. In some embodiments, the antibody-vesicle complex is created on a solid phase. In yet other embodiments, the methods further comprise releasing the vesicle from the antibody-vesicle complex. In certain embodiments, the solid phase is non-magnetic beads, magnetic beads, agarose, or sepharose. In other embodiments, the vesicle is released by exposing the antibody-vesicle complex to low pH between 3.5 and 1.5. In other embodiments, the vesicle is released using a competing peptide that competes for the binding of the selection antibody used in the methods of the present invention. In yet other embodiments, the released vesicle is neutralized by adding a high pH solution. In other embodiments, the released vesicle is lysed by incubating the released vesicles with a lysis solution. In still other embodiments, the lysis solution contains inhibitors for proteases and phosphatases.

### Neurodegenerative Disorders

The present disclosure provides methods for diagnosing or prognosing a neurodegenerative disorder in a subject, identifying a subject at risk of a neurodegenerative disorder, or prescribing a therapeutic regimen or predicting benefit from therapy in a subject having a neurodegenerative disorder.

In some embodiments the neurodegenerative disorder is selected from the group consisting of:
Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease.

In some embodiments, the present disclosure enables a medical practitioner to diagnose or prognose one or more neurodegenerative disorder in a subject. In other embodiments, the present invention enables a medical practitioner to rule out or eliminate one or more neurodegenerative diseases as a diagnostic possibility. In other embodiments, the methods of the present invention allow a medical practitioner to distinguish some forms of FTD from Alzheimer's disease. In yet other embodiments, the present disclosure enables a medical practitioner to identify a subject at risk of developing a neurodegenerative disorder. In other embodiments, the present invention enables a medical practitioner to predict whether a subject will later develop a neurodegenerative disorder. In further embodiments the present disclosure enables a medical practitioner to prescribe a therapeutic regimen or predict benefit from therapy in a subject having a neurodegenerative disorder.

### Biomarkers

Biomarker levels are detected or assayed in a biological sample obtained from a subject having or at-risk of having a neurodegenerative disorder (e.g., Alzheimer's disease). In some embodiments, the biomarker is synaptophysin, synaptopodin, synaptotagmin, neurogranin, or GAP43. Other known neurodegenerative disorder biomarkers may be used in combination with the biomarkers of the present invention. Examples of such biomarkers are provided in US Patent Application Pub. No. 2015/0119278.

In some embodiments, biomarker levels of the present invention are measured by determining the gene expression of the biomarker. In other embodiments, biomarkers are detected using agents of the present invention, such as, for example antibodies specific for the biomarkers. In certain embodiments, gene expression changes are measured by determining the expression level of one or more of the genes shown in Table 1. In certain aspects, gene expression of the biomarker is determined using PCR, microarray, or sequencing. In some embodiments, the expression level of the biomarker is determined by measuring the mRNA or miRNA level of the biomarker.

One of ordinary skill in the art has several methods and devices available for the detection and analysis of the markers of the instant invention. With regard to polypeptides or proteins in patient test samples, immunoassay devices and methods are often used. These devices and methods can utilize labeled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labeled molecule.

Preferably the markers are analyzed using an immunoassay, although other methods are well known to those skilled in the art (for example, the measurement of marker RNA levels). The presence or amount of a marker is generally determined using antibodies specific for each marker and detecting specific binding. Any suitable immunoassay may be utilized, for example, enzyme- linked immunoassays (ELISA), radioimmunoassay (RIAs), competitive binding assays, planar waveguide technology, and the like. Specific immunological binding of the antibody to the marker can be detected directly or indirectly. Direct labels include fluorescent or luminescent tags, metals, dyes, radionuclides, and the like, attached to the antibody. Indirect labels include various enzymes well known in the art, such as alkaline phosphatase, horseradish peroxidase and the like.

The use of immobilized antibodies specific for the markers is also contemplated by the present invention. The antibodies could be immobilized onto a variety of solid supports, such as magnetic or chromatographic matrix particles, the surface of an assay place (such as microtiter wells), pieces of a solid substrate material (such as plastic, nylon, paper), and the like. An assay strip could be prepared by coating the antibody or a plurality of antibodies in an array on solid support. This strip could then be dipped into the test sample and then processed quickly through washes and detection steps to generate a measurable signal, such as a colored spot.

The analysis of a plurality of markers may be carried out separately or simultaneously with one test sample. Several markers may be combined into one test for efficient processing of a multiple of samples. In addition, one skilled in the art would recognize the value of testing multiple samples (for example, at successive time points) from the same individual. Such testing of serial samples will allow the identification of changes in marker levels over time. Increases or decreases in marker levels, as well as the absence of change in marker levels, would provide useful information about the disease status that includes, but is not limited to identifying the approximate time from onset of the event, the presence and amount of salvageable tissue, the appropriateness of drug therapies, the effectiveness of various therapies, identification of the severity of the event, identification of the disease severity, and identification of the patient's outcome, including risk of future events.

An assay consisting of a combination of the markers referenced in the instant invention may be constructed to provide relevant information related to differential diagnosis. Such a panel may be constructed using 1, 2, 3, 4, 5, 6, 7, 8, 9, 10, 15, 20, or more or individual markers. The analysis of a single marker or subsets of markers comprising a larger panel of markers could be carried out methods described within the instant invention to optimize clinical sensitivity or specificity in various clinical settings.

The analysis of markers could be carried out in a variety of physical formats as well. For example, the use of microtiter plates or automation could be used to facilitate the processing of large numbers of test samples. Alternatively, single sample formats could be developed to facilitate immediate treatment and diagnosis in a timely fashion, for example, in ambulatory transport or emergency room settings. Particularly useful physical formats comprise surfaces having a plurality of discrete, addressable locations for the detection of a plurality of different analytes. Such formats include protein microarrays, or "protein chips" and capillary devices.

Biomarkers of the present disclosure serve an important role in the early detection and monitoring of neurodegenerative disorders (e.g., Alzheimer's disease). Markers of such disorders are typically substances found in a bodily sample that can be measured. The measured amount can correlate to underlying disorder or disease pathophysiology, presence or absence of a neurodegenerative disorder, probability of a neurodegenerative disorder in the future. In patients receiving treatment for their condition the measured amount will also correlate with responsiveness to therapy. In some embodiments, a decrease in the level of one or more biomarkers of the present invention is indicative of a first neurodegenerative disorder. In some embodiments, the decrease in the level of one or more biomarkers and the lack of a decrease in the level of a different biomarker of the present invention is indicative of a second neurodegenerative disorder. For example, a decrease in synaptopodin levels is indicative of both frontotemporal dementia and Alzheimer's disease. Whereas a decrease in GAP43 levels is indicative of Alzheimer's disease and not FTD. Accordingly, the methods of the present invention are useful for the differential diagnosis of neurodegenerative disorders, such as, for example FTD and Alzheimer's disease.

Biomarkers of the present disclosure include biomarker is synaptophysin, synaptopodin, synaptotagmin, neurogranin, and GAP43. The synaptic proteins found in neural-derived exosomes (NDEs) at decreased levels for FTD and AD patients, as contrasted with matched cognitively normal controls, differ in synaptic localization and functions. The presynaptic vesicle protein synaptophysin binds synaptobrevin and regulates vesicle fusion and recycling (see, e.g., Daly et al. J Biol Chem 2002;277:9010-9015; Valtorta et al. Bioassays 2004;26:445-453). The synaptotagmins also are presynaptic vesicle proteins that bind calcium and thereby initiate vesicle fusion (Sudhof Annu Rev Neurosci 2004;27:509-547). The synaptic membrane protein GAP43 appears to share functions with synaptophysin and synaptotagmins (Haruta et al. Biochem J 1997;325(2):455-463). The two post-synaptic proteins synaptopodin and neurogranin regulate intracellular calcium concentration as well (Reddy et al. J Alzheimers Dis 2005;7:103-117). Several of these proteins are required for distinct synaptic functions, for example synaptotagmin for synaptic facilitation and maintenance of the readily releasable pool of synaptic vesicles (Bacaj et al. PLoS Biol 2015;13:e1002267; Jackman et al. Nature 2016;529:88-91). Mutations in human synaptophysin are recognized in X-linked intellectual disability and of synaptotagmin-1 in altered states of cognition and movement (Gordon. J Neurosci 2013;33:13695-13700; Baker et al. J Clin Invest 2015;125:1670-1678). The extent of losses of these synaptic proteins in hippocampal and cortical tissues at autopsy of patients with AD parallels decreases in the number of synapses and correlates with decreases in premortem cognitive functions (Clare et al. J Neurosci Res 2010;88:2083-2090; Reddy et al. J Alzheimers Dis 2005;7:103-117).

In some embodiments, the biomarker is measured by a method selected from the group consisting of immunohistochemistry, immunocytochemistry, immunofluorescence, immunoprecipitation, western blotting, and ELISA.

### Clinical Assay Performance

The methods of the present invention may be used in clinical assays to diagnose or prognose a neurodegenerative disorder in a subject, identify a subject at risk of a neurodegenerative disorder, and/or for prescribing a therapeutic regimen or predicting benefit from therapy in a subject having a neurodegenerative disorder. Clinical assay performance can be assessed by determining the assay's sensitivity, specificity, area under the ROC curve (AUC), accuracy, positive predictive value (PPV), and negative predictive value (NPV). Disclosed herein are assays for diagnosing or prognosing a neurodegenerative disorder in a subject, identifying a subject at risk of a neurodegenerative disorder, or for prescribing a therapeutic regimen or predicting benefit from therapy in a subject having a neurodegenerative disorder.

The clinical performance of the assay may be based on sensitivity. The sensitivity of an assay of the present invention may be at least about 40%, 45%, 50%, 55%, 60%, 65%, 70%, 75%, 80%, 85%, 90%, 95%, 99%, or 100%. The clinical performance of the assay may be based on specificity. The specificity of an assay of the present invention may be at least about 40%, 45%, 50%, 55%, 60%, 65%, 70%, 75%, 80%, 85%, 90%, 95%, 99%, or 100%. The clinical performance of the assay may be based on area under the ROC curve (AUC). The AUC of an assay of the present invention may be at least about 0.5, 0.55, 0.6, 0.65, 0.7, 0.75, 0.8, 0.85, 0.9, or 0.95. The clinical performance of the assay may be based on accuracy. The accuracy of an assay of the present invention may be at least about 40%, 45%, 50%, 55%, 60%, 65%, 70%, 75%, 80%, 85%, 90%, 95%, 99%, or 100%.

### Compositions

Compositions useful in the methods of the present invention include compositions that specifically recognize a biomarker associated with a neurodegenerative disorder, wherein the biomarker is synaptophysin, synaptopodin, synaptotagmin, neurogranin, and GAP43. In yet other embodiments, the composition is selected from the group consisting of a peptide, a nucleic acid, an antibody, and a small molecule.

In certain embodiments, the present disclosure relates to compositions that specifically detect a biomarker associated with a neurodegenerative disorder. As detailed elsewhere herein, the present disclosure is based upon the finding that synaptophysin, synaptopodin, synaptotagmin, neurogranin, and GAP43are specific biomarkers for AD and other neurodegenerative disorders. In some embodiments, the compositions of the present disclosure specifically bind to and detect synaptophysin, synaptopodin, synaptotagmin, neurogranin, and GAP43.

In some embodiments, the composition comprises an antibody, where the antibody specifically binds to a biomarker or vesicles of the disclosure. The term "antibody" as used herein and further discussed below is intended to include fragments thereof which are also specifically reactive with a biomarker or vesicle (e.g., exosome). Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. Antigen-binding portions may also be produced by recombinant DNA techniques or by enzymatic or chemical cleavage of intact antibodies. Antigen-binding portions include, inter alia, Fab, Fab', F(ab')₂, Fv, dAb, and complementarity determining region (CDR) fragments, single-chain antibodies (scFv), single domain antibodies, bispecific antibodies, chimeric antibodies, humanized antibodies, diabodies and polypeptides that contain at least a portion of an immunoglobulin that is sufficient to confer specific antigen binding to the polypeptide. In certain embodiments, the antibody further comprises a label attached thereto and able to be detected (e.g., the label can be a radioisotope, fluorescent compound, enzyme or enzyme co-factor).

In certain embodiments, an antibody of the disclosure is a monoclonal antibody, and in certain embodiments, the disclosure makes available methods for generating novel antibodies that specifically bind the biomarker or the exosome of the invention. For example, a method for generating a monoclonal antibody that specifically binds a biomarker or exosome, may comprise administering to a mouse an amount of an immunogenic composition comprising the biomarker or exosome, or fragment thereof, effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monocolonal antibody that binds specifically to the biomarker or exosome. Once obtained, a hybridoma can be propagated in a cell culture, optionally in culture conditions where the hybridoma-derived cells produce the monoclonal antibody that binds specifically to the biomarker or exosome. The monoclonal antibody may be purified from the cell culture.

The term "specifically reactive with" as used in reference to an antibody is intended to mean, as is generally understood in the art, that the antibody is sufficiently selective between the antigen of interest (e.g., a biomarker or exosome) and other antigens that are not of interest. In certain methods employing the antibody, such as therapeutic applications, a higher degree of specificity in binding may be desirable. Monoclonal antibodies generally have a greater tendency (as compared to polyclonal antibodies) to discriminate effectively between the desired antigens and cross-reacting polypeptides. One characteristic that influences the specificity of an antibody:antigen interaction is the affinity of the antibody for the antigen. Although the desired specificity may be reached with a range of different affinities, generally preferred antibodies will have an affinity (a dissociation constant) of about 10⁻⁶, 10⁻⁷, 10⁻⁸, 10⁻⁹ or less.

Antibodies can be generated to bind specifically to an epitope of an exosome or a biomarker of the present invention, including, for example, neuron-derived exosomes, synaptophysin, synaptopodin, synaptotagmin, neurogranin, and GAP43.

In addition, the techniques used to screen antibodies in order to identify a desirable antibody may influence the properties of the antibody obtained. A variety of different techniques are available for testing interaction between antibodies and antigens to identify particularly desirable antibodies. Such techniques include ELISAs, surface plasmon resonance binding assays (e.g., the Biacore binding assay, Biacore AB, Uppsala, Sweden), sandwich assays (e.g., the paramagnetic bead system of IGEN International, Inc., Gaithersburg, Md.), western blots, immunoprecipitation assays, immunocytochemistry, and immunohistochemistry.

In some embodiments, the present disclosure relates to compositions used for treating or preventing a neurodegenerative disorder. As detailed elsewhere herein, the present disclosure is based upon the findings that synaptophysin, synaptopodin, synaptotagmin, neurogranin, and GAP43 levels are implicated in the pathology of a variety of neurodegenerative disorders, such as, for example, Alzheimer's disease. Therefore, in one embodiment, the present disclosure provides compositions that prevent decreases in synaptophysin, synaptopodin, synaptotagmin, neurogranin, and GAP43 levels. In one embodiment, the compositions increase synaptophysin, synaptopodin, synaptotagmin, neurogranin, and GAP43 levels.

In certain embodiments, the present disclosure relates to compositions for lysing vesicles (e.g., exosomes) in biological samples obtained from a subject. Lytic agents useful in the methods of the present invention include: RIPA buffer; Tris-HCl (pH 6.8); glycerol; SDS; 2-mercaptoethanol; Triton-X 100; M-PER Reagent; T-PER solution; and CHAPS. Lytic agents may be incubated with biological samples to disrupt the membrane of the vesicles of the present invention and release vesicle cargo (e.g., exosomal proteins) for subsequent analysis.

### Methods of Treatment

The present disclosure provides methods of treating a neurodegenerative disorder in a subject, comprising administering to the subject an effective amount of a composition, wherein the composition increases or maintains the level of synaptophysin, synaptopodin, synaptotagmin, neurogranin, and GAP43 in the subject. In yet other embodiments, the composition prevents decreases in synaptophysin, synaptopodin, synaptotagmin, neurogranin, and GAP43 levels. In other embodiments, the present disclosure provides methods of treating a neurodegenerative disorder in a subject, comprising administering to the subject an effective amount of a composition, wherein the composition normalizes the level of synaptophysin, synaptopodin, synaptotagmin, neurogranin, and GAP43 to a reference level.

### Kits

Another aspect of the disclosure encompasses kits for detecting or monitoring a neurodegenerative disorder in a subject. A variety of kits having different components are contemplated by the current disclosure. Generally speaking, the kit will include the means for quantifying one or more biomarkers in a subject. In another embodiment, the kit will include means for collecting a biological sample, means for quantifying one or more biomarkers in the biological sample, and instructions for use of the kit contents. In certain embodiments, the kit comprises a means for enriching or isolating exosomes in a biological sample. In further aspects, the means for enriching or isolating exosomes comprises reagents necessary to enrich or isolate exosomes from a biological sample. In certain aspects, the kit comprises a means for quantifying the amount of a biomarker. In further aspects, the means for quantifying the amount of a biomarker comprises reagents necessary to detect the amount of a biomarker.

**Table 1**

| **Gene** | **Entrez Gene Name** | **Location** |
|---|---|---|
| Synaptophysin | SYP | Chromosome X, NC_000023.11 (49187812..49200202, complement) |
| Synaptopodin | SYNPO | Chromosome 5, NC_000005.10 (150586037..150659230) |
| Synaptotagmin 1 | SYT1 | Chromosome 12, NC_000012.12 (78863993..79452008) |
| Neurogranin | NRGN | Chromosome 11, NC_000011.10 (124739933..124747206) |
| Human Growth Associated Protein 43 | GAP43 | NC_000004.12 (56907876..56935845) |

These and other embodiments of the present invention will readily occur to those of ordinary skill in the art in view of the disclosure herein.

### EXAMPLES

The invention will be further understood by reference to the following examples, which are intended to be purely exemplary of the invention. These examples are provided solely to illustrate the claimed invention.

### Example 1: Neuron-Derived Plasma Exosomal Synaptic Protein Levels in Human Subjects with Neurodegenerative Disorders

Levels of synaptophysin, synaptopodin, synaptotagmin, neurogranin, and human growth associated protein 43 (GAP43) proteins were assayed in human subjects with neurodegenerative disorders as follows. Venous blood was collected from control subjects (AC, n=12; FTC, n=16), subjects with Alzheimer's disease (AD, n=12), and subjects with frontotemporal dementia (FTD, n=16). The diagnosis of AD and FTD was established by standard clinical and laboratory criteria.

For blood collection, 10 ml of venous blood were drawn into 0.5 ml of saline with 100 U/ml of heparin, incubated for 10 min at room temperature and centrifuged for 15 min at 1500 g. Plasmas were stored in 0.5 ml aliquots at -80°C. CSF levels of P-T181-tau and Aβ1-42 were quantified by Luminex xMAP technology using Innogenetics INNO-BIA Alz Bio3 kits.

For plasma, 250 ul received 0.1 ml of thromboplastin-D (Fisher Scientific, Inc., Hanover Park, IL) followed by addition of calcium- and magnesium-free Dulbecco's balanced salt solution with protease inhibitor cocktail (Roche Applied Sciences, Inc., Indianapolis, IN) and phosphatase inhibitor cocktail (Pierce Halt, Thermo Scientific, Inc., Rockford, IL).

After centrifugation at 3,000 x g for 30 min at 4°C, supernates were incubated with ExoQuick exosome precipitation solution (EXOQ; System Biosciences, Inc., Mountainview, CA), and the resultant suspensions were centrifuged at 1,500 x g for 30 min at 4°C. Each pellet was re-suspended in 350 µl of distilled water with inhibitor cocktails for immunochemical enrichment of exosomes from neuronal sources.

Exosome suspensions were incubated with 1.5 µg of mouse anti-human CD171 (L1CAM neural adhesion protein) biotinylated antibody (clone 5G3, eBioscience, San Diego, CA) in 50 µL of 3% BSA followed by incubation with 10 µl of Streptavidin-Plus UltraLink resin (Pierce-Thermo Scientific, Inc.) in 40 µL of 3% BSA. After centrifugation at 400 g and removal of the supernatant, each pellet was resuspended in 100 µl of 0.05 M glycine-HCl (pH 3.0), incubated at 4°C for 10 min and centrifuged at 4°C for 10 min at 4,000 g. These supernates were transferred to new Eppendorf tubes containing ten µL of 1 M Tris-HCl (pH 8.0) and 25 µL of 10% BSA, and mixed before addition of 365 µL of M-PER mammalian protein extraction reagent (Thermo Scientific, Inc.) containing the protease and phosphatase inhibitors for storage at -80°C.

NDE proteins were quantified by ELISA kits for human synaptophysin, human growth associated protein 43 (GAP43) and the tetraspanning exosome marker CD81 (American Research Products, Inc.-Cusabio, Waltham, MA), with verification of the CD81 antigen standard curve using human purified recombinant CD81 antigen (Origene Technologies, Inc., Rockville, MD), neurogranin (American Research Products, Inc.-Cloud-Clone Corp., Waltham, MA), and human synaptopodin and synaptotagmin-like protein-2 (Biomatik Corp., Wilmington, DE) according to suppliers' directions. The mean value for all determinations of CD81 in each assay group was set at 1.00 and the relative values of CD81 for each sample used to normalize their recovery.

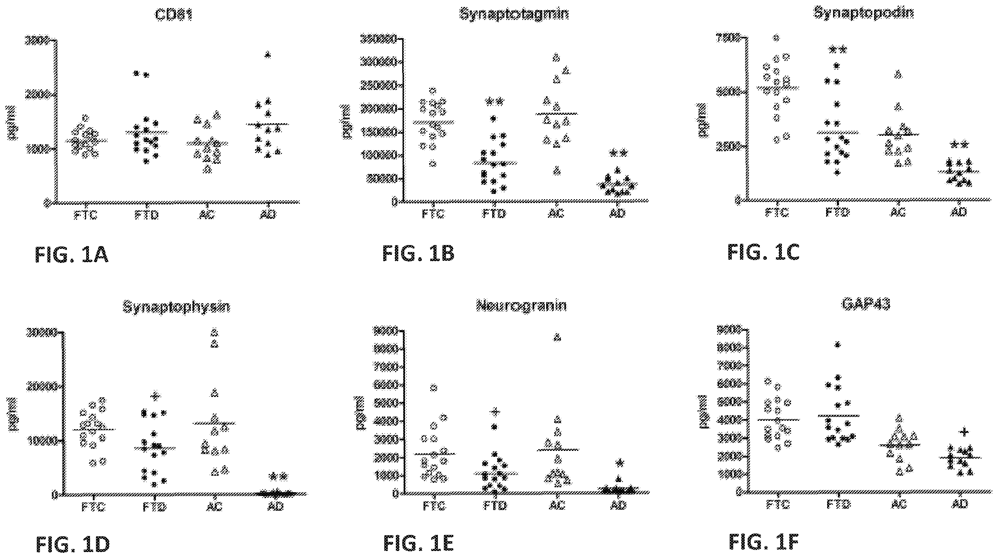

Neural exosomal levels of CD-81 showed that a similar amount of exosomes were present in samples from control (AC, FTC), AD, and FTD samples (Figure 1A). Plasma NDE levels of synaptotagmin and synaptopodin were significantly lower for the FTD and AD groups compared to control subject plasma exosomal levels (Figures 1B and1C). NDE levels of synaptophysin and neurogranin showed greater reductions for AD patients than for FTD patients when contrasted with those of their respective control groups (Figure 1D and 1E). For GAP43 levels, there was no difference between FTD and the control FTC group, and those of the AD group were only modestly significantly lower than those of the AC group (Figure 1F). The AD group had significantly lower values than the FTD group for synaptotagmin, synaptopodin, synaptophysin, neurogranin and GAP43 with respective P levels of 0.0019, 0.0003, <0.0001, 0.0052 and 0.0014. There were no differences among patient or control groups in the total levels of NDEs recovered from plasma based on content of the CD81 exosomal marker protein (Figure 1A).

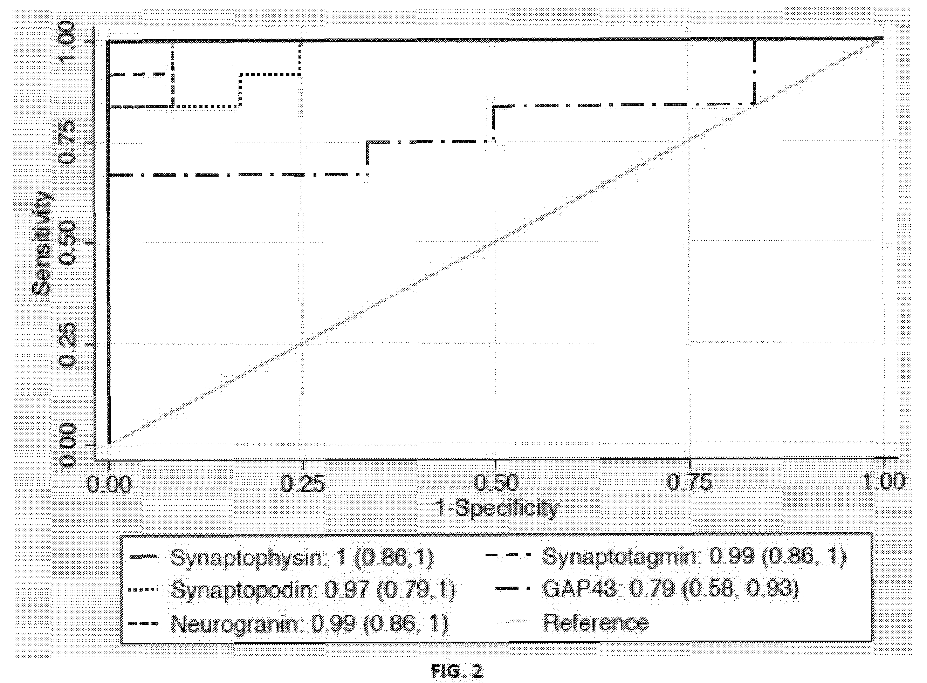

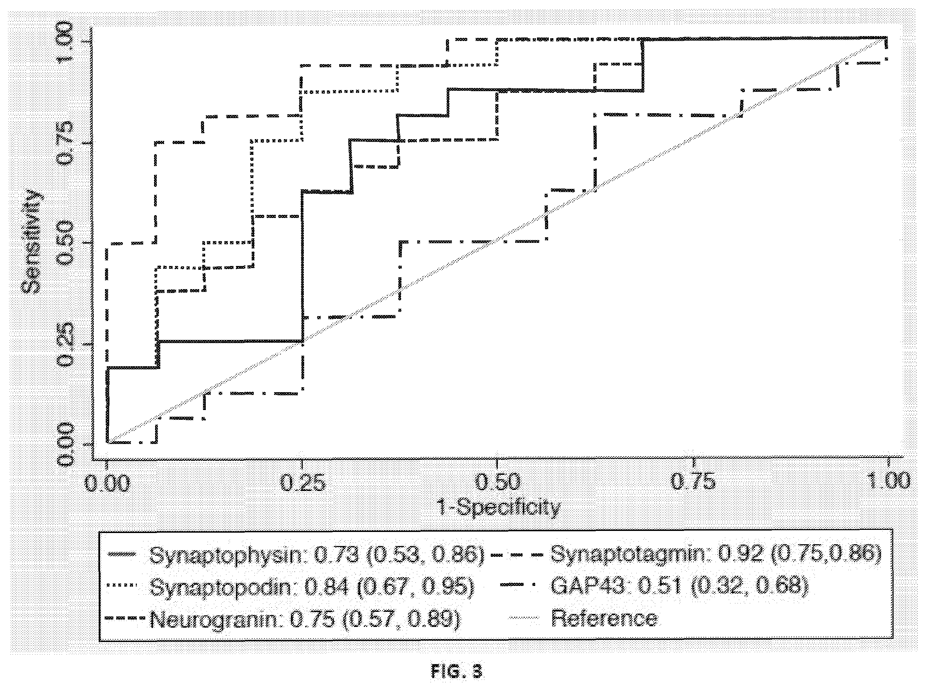

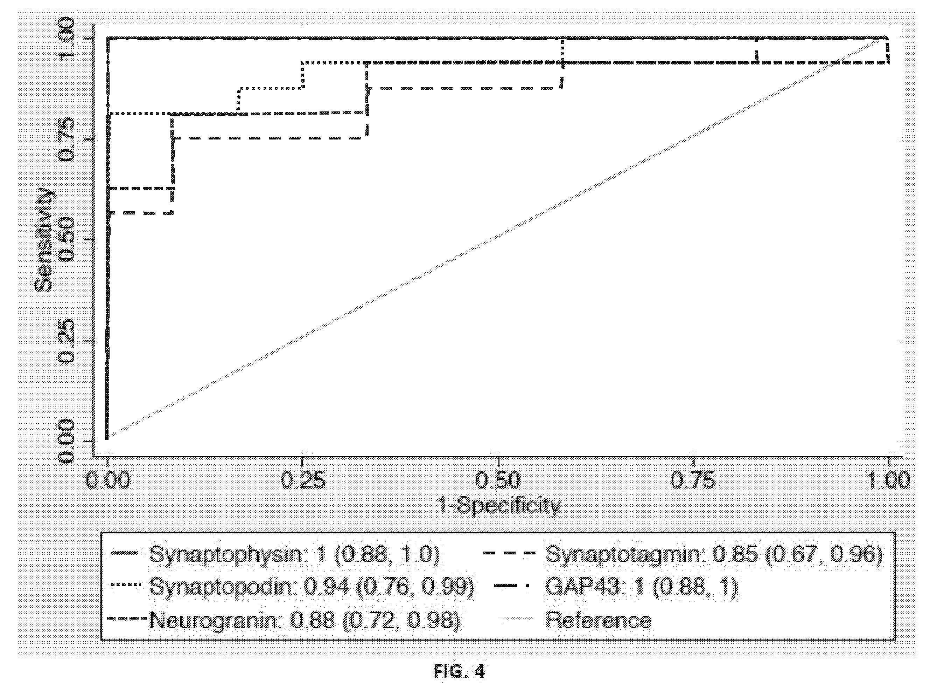

ROC plots were constructed to assess the extent of distinction between subject groups for synaptic protein levels individually and collectively (Figures 2 and 3). Discriminant analyses of matched control vs. patient groups correctly classified 91.7% of AC subjects and 100% of AD patients, with Wilk's Lambda of 0.20 and F = 14.2 (p<0.0001) (Figure 2), and 93.8% of FTC subjects and 81.3% of FTD patients, with Wilk's Lambda of 0.36 and F = 9.8 (p<0.0001) (Figure 3). Values of synaptophysin and GAP43 show no overlap between the AD and FTD groups (Figure 4). The range of synaptophysin values in AD patients was 35.2 to 617 pg/ml, whereas the range in FTD patients was 1767 to 15154 pg/ml. The range of GAP43 values in AD patients was 1079 to 2479 pg/ml, whereas the range in FTD patients was 2616 to 6286 pg/ml. ROC analyses identified a cut-off value for synaptophysin of 1767 pg/ml and for GAP43 of 2616 pg/ml that resulted in a sensitivity of 1, 95% CI=(0.79, 1), specificity of 1, 95% CI=(0.74, 1) and correct classification of 1, 95% CI=(0.88, 1) for both exosomal proteins. When considered collectively, ROC analyses of the remaining proteins synaptotagmin, synaptopodin and neurogranin all show overlapping values, but correctly classified 100% of AD patients and 75% of FTD patients, with Wilk's Lambda of 0.44 and F = 10.3 (p=0.0002)(Figure 4).

These results showed that neuron-derived plasma exosomal levels of synaptotagmin, synaptopodin, synaptophysin, neurogranin and GAP43are useful for identifying subjects with neurodegenerative disorders. These results further indicated that methods and synaptic protein biomarkers of the present invention are useful for diagnosing Alzheimer's disease and other neurodegenerative disorders.

### Example 2: Neuron-Derived Plasma Exosomal Synaptic Protein Levels Predict Development of Neurodegenerative Disorders in Human Subjects

Neuron-derived exosomal levels of synaptic proteins were assayed in human subjects as follows. Ten milliliters of venous blood were collected from subjects with dementia from AD (n=9) and from subjects with dementia from FTD (n=10) at two time-points: first when cognitively intact (AD₁, FTD₁) and again one to ten years later after diagnosis of dementia (AD₂ and FTD₂). Blood samples were processed and exosomes were isolated as described in Example 1 above. Neuron-derived exosomsal proteins synaptotagmin, synaptopodin, synaptophysin, neurogranin and GAP43were quantified by ELISA kits as described in Example 1.

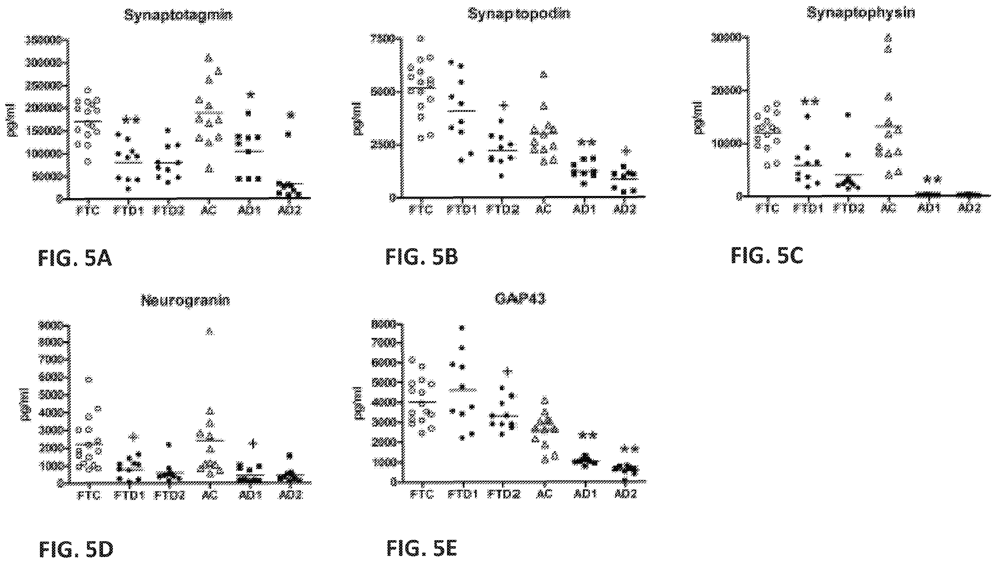

As shown in Figures 5A, 5C, and 5D, levels of synaptotagmin, synaptophysin and neurogranin for the FTD₁ and AD₁ patients were lower than for their respective control groups. However, progression of these decreases with declining cognition was observed only for the AD₂ patients. For synaptopodin and GAP43 levels, decreases relative to those of the control group were only significant at the lower level of cognition in FTD₂, whereas decreases were significant at the AD₁ stage and declined significantly further with decreasing cognition to the AD₂ stage (Figure 5B and 5E). Despite their similar levels of diminished cognition, the AD₂ group had significantly lower values than the FTD₂ group for NDE synaptotagmin, synaptopodin, synaptophysin and GAP43 with respective P levels of 0.0175, 0.0002, 0.0004 and <0.0001, but this difference was not seen for neurogranin (Figures 5A-5C).

As the patients of group AD₁ had significantly decreased levels of all NDE synaptic proteins when they were cognitively normal, these proteins may be early biomarkers of proteinopathy in AD. In contrast, levels of only three of the NDE synaptic proteins, synaptotagmin, synaptophysin and neurogranin, but not synaptopodin or GAP43, were significantly decreased at this early proteinopathic stage for the patients of group FTD₁ despite mild cognitive losses. There were no significant differences in mean levels of any synaptic protein biomarkers between subsets of AD₂ patients with two to five year and six to ten year courses or FTD₂ patients with one to two year and six to ten year courses to moderate dementia.

These results showed that neuron-derived plasma exosomal levels of synaptic proteins are lower in subjects with neurodegenerative disorders and are useful for identifying subjects with neurodegenerative disorders , such as, for example, Alzheimer's disease. These results also showed that neuron-derived plasma exosomal levels of synaptotagmin, synaptopodin, synaptophysin, neurogranin and GAP43are lower in subjects as early as 10 years before clinical diagnosis of neurodegenerative disorders. These results further showed that assays and methods of the present invention are useful for identifying a subject at risk of a neurodegenerative disorder (e.g., Alzheimer's disease). Additionally, these results showed that the assays and methods of the present invention may be useful for early detection and determining the progression of neurodegenerative disorders. These results further indicated that methods and compositions of the present invention are useful for early diagnosis and treatment of Alzheimer's disease and other neurodegenerative disorders.

## Claims

1. An in vitro method of analyzing a sample from a subject comprising the steps of: (i) obtaining a biological sample comprising vesicles from the subject, wherein the vesicles comprise one or more biomarkers, (ii) isolating the vesicles from the biological sample and measuring the level of the one or more biomarkers, and (iii) comparing the level of the one or more biomarkers in the biological sample to a control level of the one or more biomarkers in a control biological sample, wherein the subject has been diagnosed or suspected of having Alzheimer's disease or another neurodegenerative disorder, wherein the biological sample is obtained from blood, and wherein at least one of the one or more biomarkers is synaptophysin.

2. The method of claim 1, wherein the isolating vesicles from a biological sample comprises:
contacting the biological sample with an agent under conditions wherein a vesicle present in said biological sample binds to said agent to form a vesicle-agent complex; and isolating said vesicle from said vesicle-agent complex to obtain a sample containing said vesicle, wherein the purity of vesicles present in said sample is greater than the purity of vesicles present in said biological sample.

3. The method of claim 1 or 2, wherein the vesicles are selected from the group consisting of exosomes, microparticles, microvesicles, nanosomes, extracellular vesicles, and ectosomes.

4. The method of claim 3, wherein the exosomes are selected from the group consisting of neuron-derived exosomes, astrocyte-derived exosomes, oliogodendrocyte-derived exosomes, and microglia-derived exosomes.

5. The method of any one of claims 1 to 4, wherein the level of one or more biomarkers is the protein, mRNA, or miRNA level of the one or more biomarker.

6. The method of any one of the preceding claims, wherein the neurodegenerative disorder is selected from the group consisting of: Alzheimer's disease (AD), vascular disease dementia, frontotemporal dementia (FTD), corticobasal degeneration (CBD), progressive supranuclear palsy (PSP), Lewy body dementia, tangle-predominant senile dementia, Pick's disease (PiD), argyrophilic grain disease, amyotrophic lateral sclerosis (ALS), other motor neuron diseases, Guam parkinsonism-dementia complex, FTDP-17, Lytico-Bodig disease, multiple sclerosis, traumatic brain injury (TBI), and Parkinson's disease.

7. The method of any one of the preceding claims, wherein the biological sample is selected from the group consisting of whole blood, serum, and plasma.

## Patentansprüche

1. In-vitro-Verfahren zum Analysieren einer Probe aus einem Individuum, umfassend die folgenden Schritte: (i) Erhalten einer biologischen Probe, die Vesikel aus dem Individuum umfasst, wobei die Vesikel einen oder mehrere Biomarker umfassen, (ii) Isolieren der Vesikel aus der biologischen Probe und Messen der Spiegel der einen oder mehreren Biomarker und (iii) Vergleichen der Spiegel der einen oder mehreren Biomarker in der biologischen Probe zu einem Kontrollspiegel der einen oder mehreren Biomarker in einer biologischen Kontrollprobe, wobei das Individuum mit Morbus Alzheimer oder einer anderen neurodegenerativen Erkrankung diagnostiziert wurde oder im Verdacht steht, Morbus Alzheimer oder eine andere neurodegenerative Erkrankung aufzuweisen, wobei die biologische Probe aus Blut erhalten wurde und wobei es sich bei mindestens einem der Biomarker um Synaptophysin handelt.

2. Verfahren nach Anspruch 1, wobei das Isolieren der Vesikel aus einer biologischen Probe Folgendes umfasst: Inkontaktbringen der biologischen Probe mit einem Mittel unter Bedingungen, unter denen ein in der biologischen Probe vorhandenes Vesikel an das Mittel zur Bildung eines Vesikel-Mittel-Komplexes bindet, und Isolieren des Vesikels aus dem Vesikel-Mittel-Komplex zum Erhalt einer das Vesikel enthaltenden Probe, wobei die Reinheit der in der Probe vorhandenen Vesikel größer als die Reinheit der in der biologischen Probe vorhandenen Vesikel ist.

3. Verfahren nach Anspruch 1 oder 2, wobei die Vesikel aus der Gruppe bestehend aus Exosomen, Mikropartikeln, Mikrovesikeln, Nanosomen, extrazellulären Vesikeln und Ektosomen ausgewählt sind.

4. Verfahren nach Anspruch 3, wobei die Exosome aus der Gruppe bestehend aus aus Neuronen stammenden Exosomen, aus Astrozyten stammenden Exosomen, aus Oligodendrozyten stammenden Exosomen und aus Mikroglia stammenden Exosomen ausgewählt sind.

5. Verfahren nach einem der Ansprüche 1 bis 4, wobei es sich bei dem Spiegel der einen oder mehreren Biomarker um den Protein-, mRNA- oder miRNA-Spiegel der einen oder mehreren Biomarker handelt.

6. Verfahren nach einem der vorhergehenden Ansprüche, wobei die neurodegenerative Erkrankung aus der Gruppe bestehend aus den Folgenden ausgewählt ist: Morbus Alzheimer (AD= Alzheimer's disease), vaskulärer Demenz, frontotemporaler Demenz (FTD), kortikobasaler Degeneration (CBD), progressiver supranukleärer Lähmung (PSP= progressive supranuclear palsy), Demenz mit Lewy-Körperchen, seniler Demenz überwiegend mit Fibrillen, Morbus Pick (PiD= Pick's disease), Silberkornkrankheit, amyotropher Lateralsklerose (ALS), anderen Motoneuronkrankheiten, Guam-Parkinson-Demenz-Komplex, FTDP-17, Lytico-Bodig-Krankheit, multipler Sklerose, traumatischer Hirnverletzung (TBI= traumatic brain injury) und Morbus Parkinson.

7. Verfahren nach einem der vorhergehenden Ansprüche, wobei die biologische Probe aus der Gruppe bestehend aus Vollblut, Serum und Plasma ausgewählt ist.

## Revendications

1. Procédé in vitro d'analyse d'un échantillon d'un sujet comprenant les étapes de : (i) l'obtention d'un échantillon biologique comprenant des vésicules du sujet, les vésicules comprenant un ou plusieurs biomarqueurs, (ii) l'isolement des vésicules à partir de l'échantillon biologique et la mesure du taux du ou des biomarqueurs, et (iii) la comparaison du taux du ou des biomarqueurs dans l'échantillon biologique à un niveau témoin du ou des biomarqueurs dans un échantillon biologique témoin, le sujet ayant été diagnostiqué ou suspecté d'être atteint de la maladie d'Alzheimer ou d'un autre trouble neurodégénératif, l'échantillon biologique étant obtenu à partir de sang, et au moins un du ou des biomarqueurs étant la synaptophysine.

2. Procédé selon la revendication 1, l'isolement des vésicules à partir d'un échantillon biologique comprenant :
la mise en contact de l'échantillon biologique avec un agent dans des conditions dans lesquelles une vésicule présente dans ledit échantillon biologique se lie audit agent pour former un complexe vésicule-agent ; et
l'isolement de ladite vésicule dudit complexe vésicule-agent pour obtenir un échantillon contenant ladite vésicule, la pureté des vésicules présentes dans ledit échantillon étant supérieure à la pureté des vésicules présentes dans ledit échantillon biologique.

3. Procédé selon la revendication 1 ou 2, les vésicules étant choisies dans le groupe constitué par les exosomes, les microparticules, les microvésicules, les nanosomes, les vésicules extracellulaires et les ectosomes.

4. Procédé selon la revendication 3, les exosomes étant choisis dans le groupe constitué des exosomes dérivés de neurones, des exosomes dérivés d'astrocytes, des exosomes dérivés d'oliogodendrocytes et des exosomes dérivés de microglie.

5. Procédé selon l'une quelconque des revendications 1 à 4, le taux d'un ou plusieurs biomarqueurs étant le taux de protéine, d'ARNm ou de miARN du ou des biomarqueurs.

6. Procédé selon l'une quelconque des revendications précédentes, le trouble neurodégénératif étant choisi dans le groupe constitué par : la maladie d'Alzheimer (MA), la démence vasculaire, la démence frontotemporale (DFT), la dégénérescence corticobasale (DCB), la paralysie supranucléaire progressive (PSP), la démence à corps de Lewy, la démence sénile à prédominance d'enchevêtrement, la maladie de Pick (DIP), la maladie des grains argyrophiles, la sclérose latérale amyotrophique (SLA), d'autres maladies des motoneurones, le complexe parkinsonisme de Guamdémence, la FTDP-17, le syndrome de Guam, la sclérose en plaques, le traumatisme crânien (TBI) et la maladie de Parkinson.

7. Procédé selon l'une quelconque des revendications précédentes, l'échantillon biologique étant choisi dans le groupe constitué de sang total, de sérum et de plasma.
